(19) Europäisches Patentamt
European Patent Office
Office européen des brevets

(11) Publication number: **0 184 287**
**A1**

(12) # EUROPEAN PATENT APPLICATION

(21) Application number: 85306428.5

(22) Date of filing: 10.09.85

(51) Int. Cl.[4]: C 07 D 213/74
C 07 D 213/57, A 61 K 31/44

(30) Priority: 11.09.84 GB 8422919

(43) Date of publication of application:
11.06.86 Bulletin 86/24

(84) Designated Contracting States:
AT BE CH DE FR GB IT LI LU NL SE

(71) Applicant: MAY & BAKER LIMITED

Dagenham Essex, RM10 7 XS(GB)

(72) Inventor: Lunt, Edward
May & Baker Limited
Dagenham Essex RM10 7XS(GB)

(72) Inventor: Saunders, Libert Clinton
May & Baker Limited
Dagenham Essex RM10 7XS(GB)

(72) Inventor: Stuttle, Keith Alfred James
May & Baker Limited
Dagenham Essex RM10 7XS(GB)

(74) Representative: Bentham, Stephen et al,
J.A. Kemp & Co. 14 South Square Gray's Inn
London WC1R 5EU(GB)

(54) **Pyridylaminoethenes.**

(57) Pyridylaminoethene compounds of the formula:

$$R^1NHCH=C(CN)_2$$

wherein
$R^1$ represents a 5-halogenopyrid-2-yl group, possess antiarthritic properties.

EP 0 184 287 A1

Croydon Printing Company Ltd.

## DESCRIPTION

## "PYRIDYLAMINOETHENES"

This invention relates to new therapeutically useful pyridylaminoethene derivatives, to a process for their preparation, to pharmaceutical compositions containing them, and to their use as pharmaceuticals.

The new pyridylaminoethene compounds are compounds of the general formula:-

$$R^1NHCH=C(CN)_2 \qquad I$$

(wherein $R^1$ represents a 5-halogenopyrid-2-yl, preferably a 5-chloropyrid-2-yl, 5-iodopyrid-2-yl or, more especially, 5-bromopyrid-2-yl group).

The compounds have valuable pharmacological properties, in particular properties which are indicative of utility in the treatment of arthritic disorders such as rheumatoid arthritis.

For example, from tests in mice it was calculated that 1-(5-bromopyrid-2-ylamino)-2,2-dicyanoethene, when twice administered orally, one hour before and four hours after the administration of antigen, each time at the doses shown in the following Table I, would reduce by 50% the inhibition of

migration of incubated mouse macrophage cells measured in a manner similar to that described by Likhite et al., Cellular Immunology, Vol. 5, 1972, page 377. This is a measure of antagonism or reduction of the levels of lymphokines and is indicative of utility in the treatment of arthritic patients.

TABLE 1

| Test Number | oral ED50 dose (mg/kg animal body weight) |
|---|---|
| 1 | 2 or less than 2 |
| 2 | less than 10 |
| 3 | approximately 6 |

Furthermore, in laboratory tests, the compounds have been shown to inhibit the deterioration of joints in the limbs of cavies and rats. These results are particularly important because compounds currently employed in the treatment of arthritic disorders are primarily antiinflammatories and do not possess the said ability to inhibit joint deterioration.

The beneficial properties of the compounds of formula I are enhanced by the fact that they have only very low mammalian toxicity.

The compounds of formula I may be prepared by the application or adaptation of known methods.

Thus as a feature of the present invention, the compounds of formula I are prepared by the reaction of compounds of the general formula:-

$$R^1\text{-}NH_2 \qquad \text{II}$$

(wherein $R^1$ is as hereinbefore defined) with compounds of the general formula:-

$$R^2OCH{=}C(CN)_2 \qquad \text{III}$$

(wherein $R^2$ represents an alkyl group of 1 to 5 carbon atoms, preferably with a straight chain, e.g. ethyl), generally at temperatures between 0° and 250°C, preferably at or near room temperature, optionally in the presence of an inert solvent. Suitable inert solvents include aromatic hydrocarbons,

e.g. toluene, aliphatic ethers, aliphatic nitriles, aliphatic amides, e.g. N,N-dimethylacetamide, and, more especially, aliphatic alcohols, e.g. ethanol.

Compounds of formula III may be prepared by the application or adaptation of known methods.

By the term "known methods" as used in this specification is meant methods heretofore used or described in the literature.

The following Examples illustrate the preparation of the compounds of the present invention.

EXAMPLE 1

A mixture of 2-amino-5-bromopyridine (3.5g), 1-ethoxy-2,2-dicyanoethene (2.5g) and ethanol (50ml) was left to stand at room temperature overnight. The resulting red solid was filtered off, washed with ethanol and recrystallised from ethanol, to give 1-(5-bromopyrid-2-ylamino)-2,2-dicyanoethene (3.8g), in the form of dark red crystals, m.p. 222-224°C.

EXAMPLE 2

A mixture of 2-amino-5-bromopyridine (2.1g), 1-ethoxy-2,2-dicyanoethene (1.5g) and N,N-dimethylacetamide (30ml) was heated at reflux for 3.5 hours. It was then poured onto ice and the resulting solid was filtered off, dried, subjected to medium pressure chromatography on silica gel using a mixture of chloroform and ethyl acetate (4:1 v/v) as eluant, and

recrystallised from ethanol, to give 1-(5-bromopyrid-2-ylamino)-2,2-dicyanoethene (0.35g) in the form of an orange powder, m.p. 220-221°C.

The present invention includes within its scope pharmaceutical compositions which comprise at least one compound of formula I in association with a pharmaceutically acceptable carrier or coating. In clinical practice the compositions of the present invention will normally be administered orally or rectally, or parenterally, for example topically or intraarticularly.

Solid compositions for oral administration include compressed tablets, pills, dispersible powders, and granules. In such solid compositions one or more of the active compounds is mixed with at least one inert diluent such as calcium carbonate, potato starch, alginic acid, or lactose. The compositions may also comprise, as is normal practice, additional substances other than inert diluents, e.g. lubricating agents, such as magnesium stearate. Liquid compositions for oral administration include pharmaceutically acceptable emulsions, solutions, suspensions, and elixirs containing inert diluents commonly used in the art, such as water and liquid paraffin. Besides inert diluents such compositions may also comprise adjuvants, such as wetting and

suspending agents, and sweetening, flavouring, perfuming and preserving agents.

The compositions according to the invention, for oral administration, also include capsules of absorbable material such as gelatin containing one or more of the active compounds with or without the addition of diluents or excipients.

Solid compositions for rectal administration include suppositories formulated in manner known *per se* and containing one or more of the active compounds.

Preparations according to the invention for parenteral administration include sterile aqueous or non-aqueous solutions, suspensions, or emulsions. Examples of non-aqueous solvents or suspending media are propylene glycol, vegetable oils such as olive oil, and injectable organic esters such as ethyl oleate. These compositions may also contain adjuvants such as preserving, wetting, emulsifying and dispersing agents. They may be sterilised, for example, by filtration through a bacteria-retaining filter, by incorporation of sterilising agents in the compositions or by irradiation. They may also be manufactured in the form of sterile solid compositions, which can be dissolved in a sterile injectable medium immediately before use. As well as

the more customary intravenous and intramuscular routes, the compositions may be administered by intraarticular injection.

Compositions in the form of solutions or suspensions, if desired together with additives as described above, in water or in vegetable or other greases, paraffin or other waxes, or lacquers or creams or lotions, to be applied topically, for example to the skin area around an affected joint to relieve arthritis, are also included in the invention. They may also include additives such as nicotinamide to assist absorption.

The percentages of active ingredient in the compositions of the invention may be varied, it being necessary that they should constitute a proportion such that a suitable dosage for the desired antiarthritic effect shall be obtained. Obviously several unit dosage forms may be administered at about the same time. Generally the compositions should contain 0.1% to 80% by weight of active ingredient, especially when in tablet form.

The dose employed depends upon the desired antiarthritic effect, the route of administration and the duration of the treatment. In the adult, the doses are generally between 0.01 and 100mg (preferably

orally between 0.1 and 10mg, or intramuscularly, intravenously or intraarticularly between 0.01 and 10mg) of the compounds of formula I per kg body weight per day.

The compounds of formula I may be administered each day or, according to the wishes of the medical practitioner, less often, e.g. weekly.

The present invention provides a method of treating arthritic disorders in man which comprises administering to the patient an amount of a compound of formula I sufficient to combat an arthritic disorder.

The following Composition Example illustrates pharmaceutical compositions according to the present invention.

## COMPOSITION EXAMPLE 1

Capsules for oral administration were made up in the usual manner by filling No. 2 size gelatin capsules each with 155 mg of the following composition:-

| | |
|---|---|
| 1-(5-bromopyrid-2-ylamino)-2,2-dicyanoethene | 50 mg |
| potato starch | 100 mg |
| magnesium stearate | 2.5 mg |
| Aerosil | 2.5 mg |

Similar compositions can be prepared by the use of any of the other compounds of formula I.

## CLAIMS

1. A pyridylaminoethene derivative of the general formula:

$$R^1NHCH=C(CN)_2 \qquad I$$

wherein $R^1$ represents a 5-halogenopyrid-2-yl group.

2. 1-(5-Bromopyrid-2-ylamino)-2,2-dicyanoethene.

3. A process for the preparation of a compound of the general formula:

$$R^1NHCH=C(CN)_2 \qquad I$$

wherein $R^1$ represents a 5-halogenopyrid-2-yl group, which comprises the reaction of a compound of the general formula:

$$R^1-NH_2 \qquad II$$

(wherein $R^1$ is as defined in claim 1) with a compound of the general formula:

$$R^2OCH=C(CN)_2 \qquad III$$

(wherein $R^2$ represents an alkyl group of 1 to 5 carbon atoms).

4. A process according to claim 3 in which the reaction of the compound of general formula II with the compound of general formula III is carried out at or near room temperature, optionally in the presence of an inert solvent.

5. A pharmaceutical composition which comprises, as active ingredient, a pyridylaminoethene derivative according to claim 1 in association with a pharmaceutically acceptable

carrier or coating.

6. A pyridylaminoethene derivative according to claim 1, for use in the manufacture of a pharmaceutical composition for the treatment of arthritic disorders.

CLAIMS (AT)

1. A process for the preparation of a compound of the general formula:

$$R^1NHCH=C(CN)_2 \qquad I$$

wherein $R^1$ represents a 5-halogenopyrid-2-yl group, which comprises the reaction of a compound of the general formula:

$$R^1-NH_2 \qquad II$$

(wherein $R^1$ is as hereinbefore defined) with a compound of the general formula:

$$R^2OCH=C(CN)_2 \qquad III$$

(wherein $R^2$ represents an alkyl group of 1 to 5 carbon atoms).

2. A process according to claim 1 in which the reaction of the compound of general formula II with the compound of general formula III is carried out at or near room temperature, optionally in the presence of an inert solvent.

3. A process according to claim 1 or 2 in which $R^1$ represents a 5-bromopyrid-2-yl group.

4. A pyridylaminoethene derivative of general formula I depicted in claim 1 wherein $R^1$ is as defined in claim 1, for use in the manufacture of a pharmaceutical composition for the treatment of arthritic disorders.

European Patent Office

# EUROPEAN SEARCH REPORT

0184237
Application number
EP 85 30 6428

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 80, no 21, 27th May 1984, page 416, column 2, abstract no. 120707j, Columbus, Ohio, US; Y. OKAMOTO et al.: "Reaction of ethoxymethylenemalonitrile with 2-aminopyridines", & CHEM. PHARM. BULL. 1974, 22(2), 243-247 | 1,3 | C 07 D 213/74<br>C 07 D 213/57<br>A 61 K 31/44 |
| A | CHEMICAL ABSTRACTS, vol. 84, no. 21, 24th May 1976, page 536, column 2, abstract no. 150510q, Columbus, Ohio, US; & JP - A - 50 105 668 (KOHJIN CO., LTD.) 20-08-1975 | 1,3,4 | |
| A | ACTA CHEMICA SCANDINAVICA, vol. B 31, no. 3, 1977, pages 235-238, Helsinki, FL; O. CEDER et al.: " Synthesis of the 1,3,4-Triaza- and 1,4-Diazacycl(3.3.3)azine systems" * page 237, right hand column, lines 27-40 * | 1,3,4 | TECHNICAL FIELDS SEARCHED (Int. Cl.4)<br>C 07 D 213/00<br>A 61 K 31/00 |
| A | FR-A-2 376 661 (CIBA-GEIGY AG) | | |

The present search report has been drawn up for all claims

| Place of search | Date of completion of the search | Examiner |
|---|---|---|
| BERLIN | 17-02-1986 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document